# EUROPÄISCHE PATENTANMELDUNG

(11) **EP 0 666 319 A1**
(43) Veröffentlichungstag der Anmeldung: **09.08.1995**
(21) Anmeldenummer: 95101263.2
(22) Anmeldetag: 31.01.1995
(51) Int. Cl.: C12P 7/40, C12P 3/00

(54) **Verfahren zur Gewinnung von Wasserstoff und Carbonsäuren aus Biomassenhydrolysat**

(30) Priorität: 04.02.1994 DE 4403391
(71) Anmelder: Thyssen Still Otto Anlagentechnik GmbH, D-44789 Bochum (DE)
(72) Erfinder: Tippmer, Kurt, D-45665 Recklinghausen (DE); Tippmer, Stefanie, D-45665 Recklinghausen (DE)
(74) Vertreter: Dahlkamp, Heinrich-Leo

(57) **Zusammenfassung**

Die Erfindung bezieht sich auf ein Verfahren zur Gewinnung von Wasserstoff (14) und Carbonsäuren (15) aus Biomassenhydrolysat (1), das bei der Temperaturdruckhydrolyse (TDH) von biogenen pflanzlichen oder mikrobiologischen Massen anfällt.

Erfindungsgemäß wird vorgeschlagen, daß das Hydrolysat einem anaeroben mikrobiologischen Stoffumwandlungsprozeß unter Anwesenheit von Clostridien unterzogen wird, der dabei freiwerdende Wasserstoff in einem Reaktor mit Hilfe einer hydrophoben Porenmembrane entzogen wird und die entstehenden Carbonsäuren und gegebenenfalls Alkohole zwecks Gleichgewichtseinstellung ebenfalls aus dem Hydrolysat entfernt werden.

## Beschreibung

Die Erfindung bezieht sich auf ein Verfahren zur Gewinnung von Wasserstoff und Carbonsäuren aus Biomassenhydrolysat, das bei der Temperaturdruckhydrolyse (TDH) von biogenen Massen anfällt.

Aus der nachveröffentlichten P 43 33 468.7 ist ein Verfahren zur Behandlung von biogenen Restmassen, insbesondere von bei der biologischen Abwasserreinigung anfallenden Klärschlämmen, Gülle, sonstigen mikrobiologischen und nachwachsenden Biomassen sowie von biogenen Restmüllfraktionen bekannt, bei dem die biogenen Massen sowohl einer temperaturaktivierten Flüssigphasenhydrolyse (TDH) als auch einer anaeroben Fermentation unterzogen werden, wobei im wesentlichen ein Hydrolysat mit den wasserlöslichen Spaltprodukten, ein zur Verbrennung nutzbares Gas, eine weitgehend von organischen Anteilen und Schadstoffen befreite verwertbare oder deponierfähige Restmasse und eine separate Schwermetallfraktion anfallen.

Das Verfahren der Flüssigphasenhydrolyse spaltet die Biopolymere der mikrobiologischen oder pflanzlichen Biomasse in die Monomere, so daß u. a. derivater Zucker, Carbonsäuren, Alkohole und Aminosäuren entstehen, die im sogenannten Hydrolysat gelöst sind.

Dieses Hydrolysat kann als sogenannte Kohlenstoffquelle, insbesondere da das C/N-Verhältnis > 12 ist, bei verschiedenen mikrobiologischen Prozessen dienen, wie z. B. bei der
- anaerobe Faulung, Biogaserzeugung
- Denitrifikation von NO₃⁻ zu N₂
- Nährlösung für das Wachstum von Hefen wie z. B. Penicellin oder Cephalospornien
oder aber auch zur Produktion von Wasserstoff und Carbonsäuren, wie z. B. Buttersäure mittels Clostridien der Spezie Clostridium-butyricum.

Aufgabe der Erfindung soll es nun sein, ein verbessertes Verfahren zur Erzeugung von Wasserstoff und Carbonsäuren aus Biomassenhydrolysat vorzuschlagen.

Die Lösung dieser Aufgabe ist im Kennzeichen des Anspruches 1 wiedergegeben. Die Ansprüche 2 bis 6 enthalten ergänzende Verfahrensmerkmale.

Das Biomassenhydrolysat bzw. das Substrat wird in einem Rohrmembranreaktor durch die hydrophoben porigen Membranrohre geleitet und der sich bei dem mikrobiologischen Stoffwechsel bildende Wasserstoff wird aufgrund eines Druckgefälles durch die Poren der hydrophoben Membranwand in den Mantelräumen abgezogen. Ein Teilstrom des direkt über dem Membranreaktor umlaufenden Substrates kann in einer Kaskade, bestehend aus einer Crossflow-Stufe zur Abtrennung der suspendierten Bakterienmasse und in einer gegebenenfalls mehrstufigen Umkehrosmose-Stufe zur Abtrennung von Carbonsäuren, Alkoholen und Glukose weiterbehandelt werden.

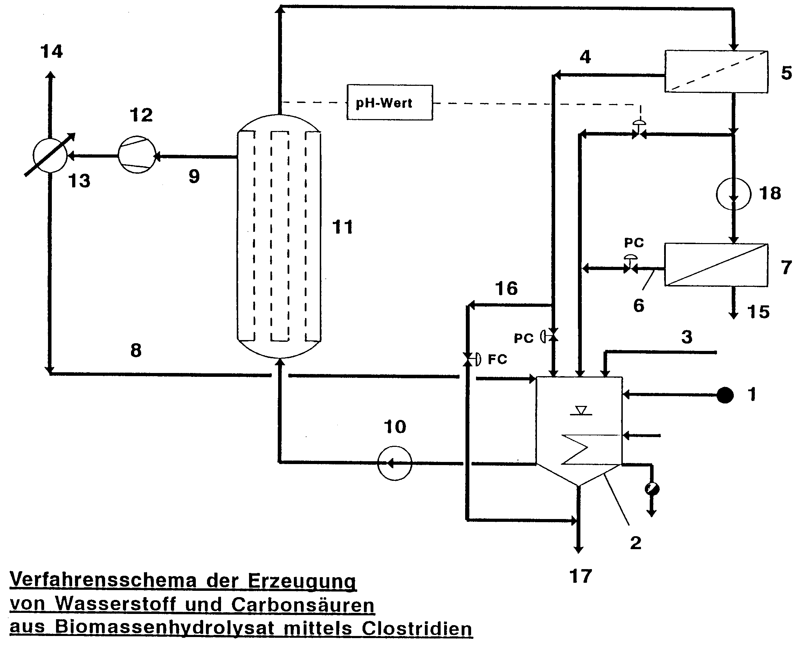

Die Erfindung wird in der Figur, die ein Verfahrensschema der Erzeugung von Wasserstoff und Carbonsäuren aus Biomassenhydrolysat mittels Clostridien enthält, beispielsweise näher erläutert.

Das gefilterte Hydrolysat (1), welches durch den hydrolytischen Prozeß dekontaminiert ist von Schwermetallen und Pestiziden, wird in den Umlaufbehälter (2) gegeben. Der Elektrolythaushalt des streng anaeroben Prozesses wird durch die ergänzende Zugabe einer Minimallösung (3) erreicht, während der pH-Wert ergänzend mittels eines basischen oder sauren Additives kontrolliert wird. Zusammen mit dem Bakterienkonzentrat (4) der MF-Stufe (5), des Retentates (6) der Umkehrosmose-Stufe (7) sowie dem Kondensat (8) der Gasphase (9) wird mit der Umlaufpumpe (10) das Hydrolysat zu dem Rohrmembran-Reaktor (11) gefördert. Der Abbau der organischen Komponenten des Hydrolysates durch die suspendierten bzw. teilweise immobilisierten Bakterien zu organischen Säuren und H₂ und CO₂ erfolgt in einer hydrophoben porösen Rohrmembrane, die auch als Oberfläche für die Bakterien-Immobilisation dient. Die Verweilzeit im Reaktionssystem beträgt mehr als 24, in der Regel 48 bis 60 Stunden bei Temperaturen von 25 bis 40 °C und pH-Werten von 3 bis 5, vorzugsweise 3,8 bis 4,2. Der bei dem Stoffwechsel freiwerdende Wasserstoff und das Kohlendioxid können zum größten Teil aufgrund des transmembranen Druckgefälles durch die Poren permeieren und so aus dem Substrat entfernt werden. Mittels einer Vakuumpumpe (12) wird die Gasphase (9) abgezogen und auf 1,2 bar (a) verdichtet. Die Vakuumpumpe (12) ist als Wasserringpumpe ausgebildet, so daß in der Verbindung mit einem Kondensator (13) wasserdampfflüchtige Produkte aus dem Gasstrom durch Absorption entfernt und als Rücklauf in das System zurückgeführt werden können. Durch die Entfernung des Wasserstoffes (14) ist den Bakterien die Möglichkeit genommen, bedingt durch gebildete Carbonsäuren oder die des Hydrolysates, durch die Bildung von Alkoholen, den pH-Wert zu senken. Der Bio-Membran-Reaktor ist mit einem nicht im Schema dargestellten inneren Kreislauf ausgebildet. Die überschüssigen Säuren, in der Hauptsache Buttersäure und Essigsäure, werden einem Teilstrom der inneren Umlauflösung über eine Umkehrosmose-Stufe (7) mit der Feedpumpe (18) bei transmembranen Drücken von 40 bis 80 bar entnommen, wobei die Trennmembrane bei einem pH-Wert von 4 bis 6 für Carbonsäuren (15) und Alkohole durchlässig ist, während andere Stoffe, z. B. Glucose, als Retentat (6) im Kreislauf geführt werden und in dem Umlauf recyceln. In einer weiteren Umkehrosmose-Stufe können z. B. die Säuren als Produkt und das Wasser getrennt werden, wobei das Wasser ebenfalls in den Prozeß recycelt wird. Dem Umkehrosmose-System ist eine Mikrofiltrationsstufe (5) nach dem Prinzip der pulsierenden tangentialen Überströmung (oder Cross-Flow-Filtration mit backflush) vorgeschaltet. Die Bakterien werden dabei als ein pumpfähiges Konzentrat (4) gewonnen und in den Prozeß recycelt. Der zwangsweise anfallende Bakterienüberschuß (16) (bis 5 % des C-Umsatzes) wird aus diesem Konzentrat abgezweigt und als Biomasse (17) wieder bei der Flüssigphasenhydrolyse in die monomeren Biobausteine gespalten.

Die Kontrolle dieses biotechnischen Prozesses erfolgt durch die folgenden Verfahrensschritte:
· Der gebildete Wasserstoff wird über die Porenmembranen aus dem Substrat entfernt, wobei der restliche Reaktionsraum ohne Membranen ein Minimum sein sollte.
· Die überschüssigen Säuren werden mittels einer Umkehrosmose-Stufe entfernt.
· Die optimale Temperatur erfolgt über eine indirekte Erwärmung der Umlauflösung.
· Anstelle eines Rohrmembranreaktors kann auch eine mehrstufige Rührkesselkaskade angewendet werden, jedoch mit geringerer H₂-Ausbeute, wobei das H₂ jeweils am Kopf eines jeden Rührkessels abgezogen wird.

Bei den Versuchen mit einer Laborapparatur wurde im Vergleich zu den bekannten Literaturwerten (Rehm, Einführung in die industrielle Mikrobiologie, Heidelberger Taschenbücher Band 84, Seite 132) festgestellt, daß sich mehr als 320 mol H₂ pro 100 mol Glukose (Literatur 235 mol/100 mol) bei einem H₂-Gehalt des Vergärungsgases von 80 Vol% (Literatur 44 Vol%) erzeugen lassen. Die reduzierte CO₂-Produktion bedingte einen Anstieg der Carbonsäuren (C-Bilanz). Andererseits wurde festgestellt, daß bei pH-Werten von 3,6 bis 3,8 die Vergärung zum Stillstand kommt. Ein kontinuierlicher Prozeß bei einem pH-Wert von 4 bis 4,2 konnte dadurch aufrechterhalten werden, daß dem Hydrolysat mittels einer Umkehrosmose-Stufe (Acetatmembran) die überschüssigen Carbonsäuren entnommen werden. Die zugegebene Nährlösung bestand aus Glukose und einer physiologischen Minimallösung. Weitere Versuche mit Hydrolysaten aus der hydrolytischen Spaltung von Biopolymeren mikrobiologischer Art (z. B. Faulschlamm) zeigten analoge Ergebnisse.

### Bezugszeichenliste

- (1): Hydrolysat
- (2): Umlaufbehälter
- (3): Minimallösung
- (4): Bakterienkonzentrat
- (5): MF-Stufe
- (6): Retentat
- (7): Umkehrosmose-Stufe
- (8): Kondensat
- (9): Gasphase
- (10): Hydrolysat
- (11): Rohrmembran-Reaktor
- (12): Vakuumpumpe
- (13): Kondensator
- (14): Wasserstoff
- (15): Carbonsäure
- (16): Bakterienüberschuß
- (17): Biomasse
- (18): Einsatzpumpe der Umkehrosmose

## Patentansprüche

1. Verfahren zur Gewinnung von Wasserstoff und Carbonsäuren aus Biomassenhydrolysat, das bei der Temperaturdruckhydrolyse (TDH) von biogenen Massen anfällt, **dadurch gekennzeichnet,** daß das Hydrolysat einem anaeroben mikrobiologischen Stoffumwandlungsprozeß unter Anwesenheit von Clostridien unterzogen wird, der dabei freiwerdende Wasserstoff in einem Reaktor mit Hilfe einer hydrophoben Porenmembrane entzogen wird und die entstehenden Carbonsäuren und gegebenenfalls Alkohole zwecks Gleichgewichtseinstellung ebenfalls aus dem Hydrolysat entfernt werden.

2. Verfahren nach Anspruch 1, **dadurch gekennzeichnet,** daß der Wasserstoff als Permeat in einem Rohrmembranreaktor bei Temperaturen von 25 bis 40°C und pH-Werten von 3 bis 5 durch die poröse hydrophobe Membran mittels Vakuum dem umlaufenden Hydrolysat entzogen wird.

3. Verfahren nach Anspruch 1 oder 2, **dadurch gekennzeichnet,** daß die überschüssige Bakterienmasse zur TDH-Stufe zwecks hydrolytischer Aufspaltung zurückgeführt wird.

4. Verfahren nach Anspruch 1, 2 oder 3, **dadurch gekennzeichnet,** daß ein bei der Vorfiltration der Umkehrosmose anfallendes Bakterienkonzentrat ganz oder teilweise zum Rohrmembranreaktor zurückgeführt wird.

5. Verfahren nach mindestens einem der Ansprüche 1 bis 4, **dadurch gekennzeichnet,** daß in dem Hydrolysat und/oder in der porösen Membranwand Kohlenstoff suspendiert bzw. immobilisiert wird.

6. Verfahren nach Anspruch 1, **dadurch gekennzeichnet,** daß die gebildeten Carbonsäuren und gegebenenfalls Alkohole in einem Umkehrosmose-System nach dem Prinzip der fraktionierenden Umkehrosmose gewonnen werden und Retentat bzw. Wasser in den Prozeß zurückgeführt wird.
